# FASCICULE DE BREVET EUROPEEN

(11) **EP 1 164 974 B1**
(45) Date de publication et mention de la délivrance du brevet: **03.08.2005**
(21) Numéro de dépôt: 00915264.6
(22) Date de dépôt: 31.03.2000
(51) Int. Cl.: A61F 2/16

(54) **IMPLANT POUR LA CORRECTION DE LA PRESBYTIE DES YEUX PHAQUES**
IMPLANTAT ZUR KORREKTUR DER PRESBYOPIE IM PHAKEN AUGE
IMPLANT FOR CORRECTING PRESBYOPIA IN PHAKIC EYES

(30) Priorité: 02.04.1999 FR 9904136
(43) Date de publication de la demande: 02.01.2002
(73) Titulaire: IOLTECH, 17180 Perigny (FR)
(72) Inventeur: BAIKOFF, Georges, 13007 Marseille (FR)
(74) Mandataire: Lewitter, Herbert
(86) Numéro de dépôt international: PCT/FR2000/000815
(87) Numéro de publication internationale: WO 2000/059406

(56) Documents cités:
- EP-A- 0 488 835
- FR-A- 2 773 318
- US-A- 4 955 902
- US-A- 5 766 245
- US-A- 5 769 890

## Description

L'invention concerne la correction de la presbytie.

Ce défaut est la perte de l'accommodation, qui touche toute la population à partir de la quarantaine. Elle se manifeste par une baisse de l'acuité visuelle de près et est généralement corrigée par le port de verres positifs convergents qui permettent de focaliser sur la rétine les images qui se formaient en arrière.

Cependant, la nécessité de porter en permanence ce type de lunettes pour les personnes emmétropes est souvent ressentie comme gênante et disgracieuse.

Pour éviter ces inconvénients, il est connu de traiter chirurgicalement la presbytie en pratiquant un remodelage de la cornée à l'aide d'un appareil à rayonnement laser connu sous la dénomination commerciale Excimer. Ce remodelage peut être fait en surface ou bien dans la profondeur du stroma cornéen, technique connue sous le nom de Lasik. Cependant, il est difficile de créer sur un tissu vivant, dont la cicatrisation est imprévisible, des courbures très précises sur des zones réduites. Les résultats de ces techniques font l'objet d'appréciations controversées.

On a plus récemment proposé une autre voie de traitement chirurgical de la presbytie par implantation sur la sclère, à l'aplomb du corps ciliaire, de segments qui ont pour fonction de retendre la zonule.

On sait par ailleurs mettre en place dans les yeux aphaques des implants de chambre postérieure placés, soit dans le sac capsulaire, soit dans le sulcus, dont la partie optique est dite multifocale car elle est traitée pour permettre une vision de près et une vision intermédiaire entre la vision de près (30 cm) et la vision de loin (à partir de 3 m).

Des implants oculaires ou intraoculaires pour la correction de la myopie forte sont connus. La partie optique de tels implants a une face concave et une périphérie épaisse. Le bord épais de la partie optique peut provoquer un phénomène d'éblouissement du bord (edge glare) lorsque des rayons à l'origine de ce phénomène ne sont plus masqués par l'iris. Le document US-A-4.955.902 vise un tel implant oculaire qui est décentré pour éliminer ce phénomène d'éblouissement chez des patients dont l'axe de la pupille est décentré. A cet effet, la partie haptique de cet implant est dissymétrique permettant de positionner ladite partie optique de manière telle que son axe focal soit décentré par rapport à l'axe géométrique de l'oeil, du côté nasal.

En revanche, l'adaptation de lentille intraoculaire pour la correction de la presbytie qui comporte une zone centrale multifocale est difficile pour de nombreux patients, ce qui empêche le développement de cette technologie pourtant avantageuse par rapport au remodelage de la cornée, en surface ou en profondeur du stroma cornéen par la technique dite Lasik.

Or, le demandeur a découvert qu'un certain nombre de cas d'inadaptation aux lentilles intraoculaires pour la correction de la presbytie était dû à un astigmatisme provoqué par le décentrage de l'axe de la zone multifocale par rapport à l'axe de la pupille.

Cependant, à la connaissance du demandeur, personne n'a jusqu'à présent imaginé ou suggéré de traiter la presbytie des yeux phaques emmétropes à l'aide d'implants disposés en chambre antérieure ou en chambre postérieure à la manière des implants utilisés couramment pour la correction des amétropies telles que la myopie.

La présente invention a pour objet de proposer un tel implant pour oeil phaque emmétrope.

Selon l'invention, il est proposé un implant oculaire pour corriger la presbytie dans un oeil phake comprenant une partie optique et une partie haptique, une partie haptique se raccordant à la partie optique, la partie optique comportant une zone centrale multifocale représentant une addition de + 1 à + 3,5 dioptries, la partie haptique étant agencée pour décentrer l'axe focal de ladite partie optique vers le côté nasal par rapport à l'axe géométrique de l'oeil, et pour assurer un décentrement de 0 à 0,75 mm de l'axe focal de la partie optique lorsque l'implant est en place dans l'oeil.

Pour une meilleure compréhension de l'invention, on va décrire ci-après deux variantes de réalisation de l'invention en relation avec les dessins annexés dans lesquels :
- la figure 1 est une vue schématique en coupe d'un implant selon l'invention mis en place dans la chambre antérieure de l'oeil,
- la figure 2 est une vue en plan de l'implant de la figure 1,
- la figure 3 est une vue schématique en coupe d'un implant selon l'invention mis en place dans la chambre postérieure de l'oeil,
- la figure 4 est une vue en plan de l'implant de la figure 3.

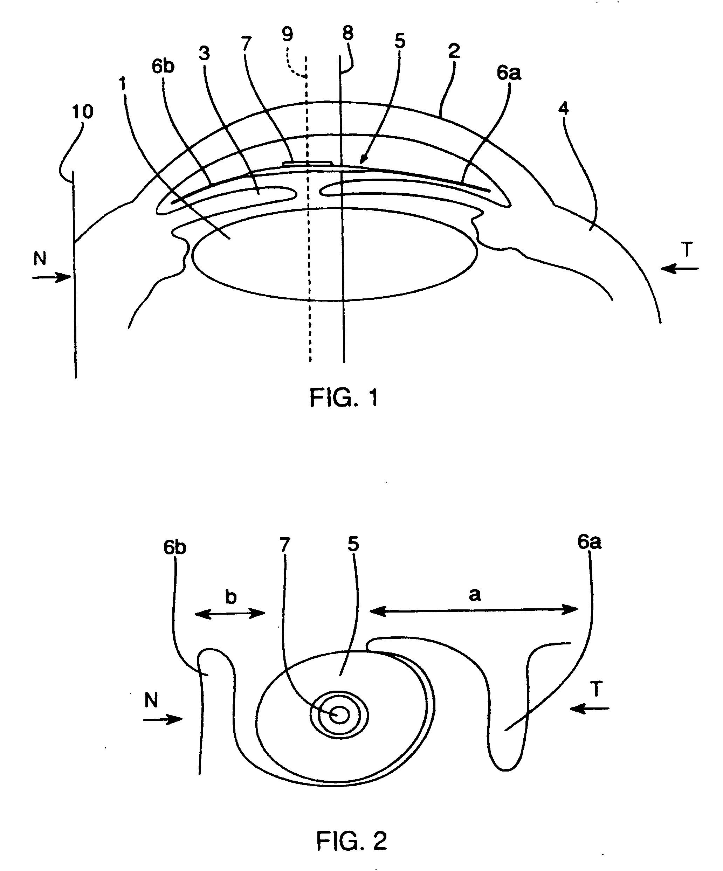

Sur la figure 1 est représentée en coupe schématique partielle la partie avant de l'oeil droit, le côté nasal étant matérialisé par la ligne 10.

Sur le schéma, 1 est le cristallin, 2 la cornée, 3 l'iris et 4 la sclère. La ligne 8 figure l'axe géométrique de l'oeil, la ligne pointillée 9 figure l'axe optique, déterminé par le centre de l'orifice pupillaire et qui est décentré du côté nasal par rapport à l'axe 8.

Les flèches N et T indiquent le côté nasal et le côté temporal de l'oeil.

Dans la chambre antérieure prenant appui dans l'angle irido-cornéen par ses anses 6a et 6b se trouve un implant selon l'invention.

Sa partie optique est constituée d'un disque 5 en matériau optique (PMMA, silicone, matériau acrylique, etc.) de puissance focale nulle et qui dans cet exemple présente en coupe une légère concavité en arrière épousant celle de l'iris 3.

Dans sa zone centrale, le disque 5 présente une zone multifocale correctrice 7 dont la puissance est calculée pour ajouter une correction de + 1 à + 3,5 dioptries correspondant à l'addition nécessaire pour pallier la presbytie.

La lentille correctrice multifocale peut être constituée de manière connue en soi par modification du rayon de courbure central sur une très petite zone de 2 à 3 mm de diamètre qui peut être reliée au disque 5 par tout moyen. Le rayon de courbure de cette lentille 7 peut être unique ou présenter des progressions pour avoir un foyer central dit progressif. Cette lentille 7 peut être également constituée par une succession de modifications concentriques du rayon de courbure alternant des zones afocales de loin et des zones focales de près, la vision de loin pouvant être placée au centre ou en périphérie de l'axe optique de la lentille. La lentille 7 peut également être constituée à la façon d'une lentille de FRESNEL avec des faces afocales et des faces surcorrigées de + 1 à + 3,5 dioptries.

A la périphérie du disque 5 constituant la partie optique de l'implant, se raccordent les anses 6a et 6b constituant sa partie haptique. On voit sur la figure 2 que la hauteur b de l'anse 6b correspondant au côté nasal de l'oeil, est plus faible que celle a de l'anse 6a opposée.

La différence de distance entre a et b est ajustée pour correspondre à la valeur du décentrement entre l'axe géométrique et l'axe optique de l'oeil à corriger. Ainsi, lorsque l'implant se trouve en place dans l'oeil, la zone multifocale sera correctement centrée sur l'axe optique 9.

En moyenne, la valeur du décentrement de l'axe optique par rapport à l'axe géométrique de l'oeil est de 0,5 mm, cependant cette valeur est susceptible de varier d'un individu à l'autre de 0 à 0,75 mm. On peut donc adapter en fonction de la morphologie de chaque oeil, le centrage de la partie multifocale.

En conséquence, l'invention couvre des implants dont les haptiques sont agencées pour avoir en place dans l'oeil une différence de hauteur comprise entre ces deux valeurs (0 à 0,75 mm).

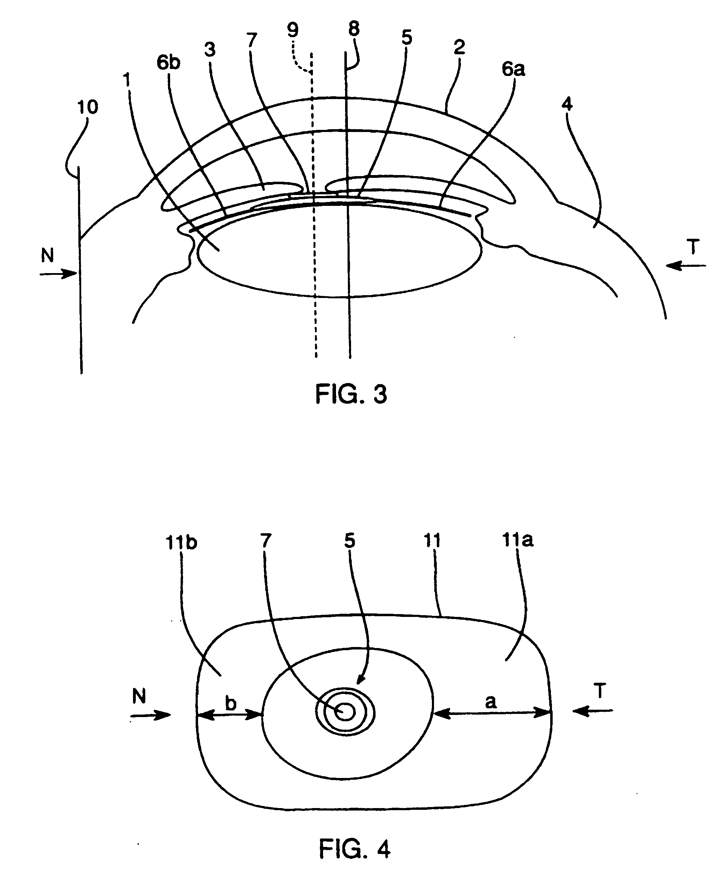

Les figures 3 et 4 représentent un implant selon l'invention destiné à être mis en place en chambre postérieure. Les références restent les mêmes que celles des figures 1 et 2 pour les parties identiques.

A la différence de l'exemple précédent, on voit que la partie haptique de l'implant est constituée non pas de deux anses diamétralement opposées, mais par un voile de forme sensiblement rectangulaire 11 solidaire de la partie optique 5 qui divise ledit voile 11 en une partie externe 11a et une partie nasale 11b. La distance b séparant le bord de la partie nasale du bord de la partie optique 5 est plus faible que celle a qui sépare la partie externe du bord correspondant de la partie optique 5. Comme précédemment, la valeur de cette différence de distance est comprise entre 0 et 0,75 mm.

Les implants selon l'invention peuvent avoir des parties haptiques de formes très variées (anses en C, anses en J, anses en Z, anneaux etc...) dès lors que cette condition de décentrement sera respectée.

L'invention est également applicable à des implants qui combinent à la fois une zone centrale multifocale pour le traitement de la vision de près et de la vision intermédiaire et une zone périphérique adaptée au traitement de la myopie, de l'astigmatisme et/ou de l'hypermétropie.

A titre d'exemple, pour un implant dont le diamètre hors tout, partie haptique comprise, sera de l'ordre de 12 à 14 mm, la partie optique aura un diamètre de l'ordre de 5 à 6 mm et la zone centrale de cette partie optique aura généralement un diamètre de 2 à 4 mm.

On peut toutefois, sans sortir du cadre de l'invention avoir un implant pour oeil emmétrope dont toute la surface de la partie optique est multifocale.

## Revendications

1. Implant oculaire pour corriger la presbytie dans un oeil phake comprenant une partie optique et une partie haptique, la partie haptique se raccordant à la partie optique, la partie optique comportant une zone centrale multifocale (7) représentant une addition de + 1 à + 3,5 dioptries, la partie haptique étant agencée pour décentrer l'axe focal de ladite partie optique vers le côté nasal par rapport à l'axe géométrique de l'oeil, et pour assurer un décentrement de 0 à 0,75 mm de l'axe focal (9) de la partie optique (5) lorsque l'implant est en place dans l'oeil.

2. Implant selon la revendication 1, **caractérisé en ce qu'**il est conformé pour être positionné dans la partie antérieure de l'oeil.

3. Implant selon la revendication 1, **caractérisé en ce qu'**il est conformé pour être mis en place dans la partie postérieure de l'oeil.

4. Implant selon l'une quelconque des revendications 1 à 3, **caractérisé en ce que** la partie optique (5) comporte en outre une zone périphérique afocale.

5. Implant selon l'une quelconque des revendications 1 à 3, **caractérisé en ce que** la partie optique (5) comporte en outre une zone périphérique focale pour corriger la myopie et/ou l'astigmatisme et/ou l'hypermétropie.

6. Implant selon l'une quelconque des revendications 1, 2, 4 et 5, **caractérisé en ce que** la partie haptique est constituée par des anses (6a, 6b).

7. Implant selon l'une quelconque des revendications 1 et 3 à 5, **caractérisé en ce que** la partie haptique est constituée par un voile de forme sensiblement rectangulaire (11).

8. Implant selon l'une quelconque des revendications 1 à 7, **caractérisé en ce que** son diamètre hors tout est compris entre 12 et 14 mm, celui de sa partie optique est compris entre 5 et 6 mm et celui de sa zone centrale multifocale est compris entre 2 et 4 mm.

## Patentansprüche

1. Okularimplantat zum Korrigieren der Presbyopie in einer Augenlinse, das einen optischen Teil und einen haptischen Teil umfasst, wobei der haptische Teil mit dem optischen Teil verbunden ist, der optische Teil eine zentrale Mehrfachbrennpunktzone (7) aufweist, die zusätzlich +1 bis +3,5 Dioptrien schafft, und der haptische Teil so beschaffen ist, dass er die Brennachse des optischen Teils in Bezug auf die geometrische Achse des Auges zur nasalen Seite dezentriert und dass er eine Dezentrierung im Bereich von 0 bis 0,75 mm der Brennachse (9) des optischen Teils (5) gewährleistet, wenn das Implantat im Auge angeordnet ist.

2. Implantat nach Anspruch 1, **dadurch gekennzeichnet, dass** es so beschaffen ist, dass es im vorderen Abschnitt des Auges positioniert werden kann.

3. Implantat nach Anspruch 1, **dadurch gekennzeichnet, dass** es so beschaffen ist, dass es im hinteren Abschnitt des Auges angeordnet werden kann.

4. Implantat nach einem der Ansprüche 1 bis 3, **dadurch gekennzeichnet, dass** der optische Teil (5) außerdem eine brennpunktlose Umfangszone aufweist.

5. Implantat nach einem der Ansprüche 1 bis 3, **dadurch gekennzeichnet, dass** der optische Teil (5) außerdem eine Brennpunkt-Umfangszone aufweist, um die Myopie und/oder den Astigmatismus und/oder die Hypermetropie zu korrigieren.

6. Implantat nach einem der Ansprüche 1, 2, 4 und 5 , **dadurch gekennzeichnet, dass** der haptische Teil durch Bügel (6a, 6b) gebildet ist.

7. Implantat nach einem der Ansprüche 1 und 3 bis 5, **dadurch gekennzeichnet, dass** der haptische Teil durch eine Schale mit im Wesentlichen rechtwinkliger Form (11) gebildet ist.

8. Implantat nach einem der Ansprüche 1 bis 7, **dadurch gekennzeichnet, dass** sein Durchmesser über alles im Bereich von 12 bis 14 mm liegt, jener seines optischen Teils im Bereich von 5 bis 6 mm liegt und jener der zentralen Mehrfachbrennpunktzone im Bereich von 2 bis 4 mm liegt.

## Claims

1. Ocular implant for correcting presbyopia in a phakic eye, comprising an optic part and a haptic part, the haptic part being joined to the optic part, the optic part having a multifocal central region (7) representing an addition from +1 to +3.5 diopters, the haptic part being adapted to position the focal axis of said optical portion off-center towards the nasal side with respect to the geometrical axis of the eye, and to ensure that the focal axis (9) of the optic part (5) is arranged from 0 to 0.75 mm off-center when the implant is in position in the eye.

2. Implant according to Claim 1, **characterized in that** it is conformed to be placed in the anterior portion of the eye.

3. Implant according to Claim 1, **characterized in that** it is conformed to be placed in the posterior portion of the eye.

4. Implant according to any one of Claims 1 to 3, **characterized in that** the optic part (5) further comprises an afocal peripheral region.

5. Implant according to any one of Claims 1 to 3, **characterized in that** the optic part (5) further includes a focal peripheral region for correcting myopia and/or astigmatism and/or hypermetropia.

6. Implant according to any one of Claims 1, 2, 4 and 5, **characterized in that** the haptic part is composed of haptic loops (6a, 6b).

7. Implant according to any one of Claims 1 and 3 to 5, **characterized in that** the haptic part is composed of a sheet (11) of substantially rectangular shape.

8. Implant according to any one of Claims 1 to 7, **characterized in that** its overall diameter is from 12 to 14 mm, that of its optic part is from 5 to 6 mm, and that of its multifocal central region is from 2 to 4 mm.
